# EUROPEAN PATENT SPECIFICATION

(11) **EP 3 905 964 B1**
(45) Date of publication and mention of the grant of the patent: **18.09.2024**
(21) Application number: 19906614.3
(22) Date of filing: 30.05.2019
(51) Int. Cl.: A61B 17/3209, A61B 17/34, A61B 17/00, A61B 17/3211, A61B 17/3205, A61B 17/32

(54) **MULTIPLE HAIR CHANNEL OPENING DEVICE**
VORRICHTUNG ZUM ÖFFNEN MEHRERER HAARKANÄLE
DISPOSITIF D'OUVERTURE D'UNE PLURALITÉ DE CANAUX CAPILLAIRES

(30) Priority: 03.01.2019 TR 201900054
(43) Date of publication of application: 10.11.2021
(73) Proprietor: Acar, Levent, 34149 Yesilköy Bakirköy/Istanbul (TR)
(72) Inventor: Acar, Levent, 34149 Yesilköy Bakirköy/Istanbul (TR)
(74) Representative: Yuce, Serfinaz Sibel
(86) International application number: PCT/TR2019/050402
(87) International publication number: WO 2020/142022

(56) References cited:
- EP-B1- 2 228 028
- WO-A1-2010/104718
- WO-A2-2008/115526
- WO-A2-2010/045158
- CN-A- 105 615 950
- US-A1- 2003 097 143
- US-A1- 2012 158 019
- US-A1- 2012 179 189
- US-A1- 2018 199 880
- US-A1- 2018 325 544
- US-A1- 2018 325 544
- US-B2- 8 211 116
- US-B2- 8 454 627

## Description

### Technical Area

The invention relates to a multi- hair channel opening device for use in hair transplantation with an angle and a direction meter, a counter and the possibility to use it in the palm.

### Infrastructure of the Invention

Hair loss is a very common problem that almost all people encounter in a certain period of their lives. Hair loss that occurs at different speeds individually starts with the removal of the forehead hairline and the dilution of the hair in the anterior and upper regions. Hair loss over time can progress to the opening of the entire hill area. These losses in hair, which constitute a very important part of the external image, cause physical problems as well as serious psychological problems.

In hair transplantation, healthy hair with non-spillage on the back and sides of the head is transported to areas where it has been lost. One of the most important stages of hair transplantation is the channel opening process. The determination of the hair direction and the determination of the hair frequency are performed by the channel-opening process. If the graft compliance is not achieved, there is no chance for hair transplantation to be successful.

Every person has a different hairline and the hair directions and angles are different. Hair grows in different angles and extend in different directions on each side of the head. The direction and angle of hair in the forehead are different as well as they are different at the top of the head and at the neck.

In order for the natural hair transplantation operation to be exact, it is necessary to open channels according to the natural hair directions and angles of the patient. This process directly affects the naturalness of the operation. The fact that the hair is opened in different directions with the opening of the channels, affects the naturalness to a great extent.

For the realization of natural hair transplantation, it is necessary to create the ideal hair line and to open the channel at the right angle and direction. In cases where the correct hair line cannot be created and the channel cannot be opened in the appropriate direction and angle, hair transplantation is no longer natural.

In the state of the art, an average of 3500 to 4500 channels is opened for a patient. This process lasts 90-120 minutes. Currently, in the manual procedure, the physician moves his hand 3500-4000 times up and down in the same plane while maintaining the same position and direction. In doing so, the physician is trying to maintain the direction and angle of the incisions on the one hand, while on the other hand he is careful not to overlap the incisions (channels). The physician counts the channels that are opened to prevent unnecessary skin damage.

In the prior art, opening larger channels may cause permanent scars. The opening of small channels, as required, can cause the roots to be damaged, dislodged and dead by force when the roots are inserted into the channels during the transplantation stage. For this reason, all channels must be opened at their correct dimensions.

Document US8454627B2 discloses systems and methods which are provided for determining the need to change and/or changing orientation of a tool relative to an object on a body in at least partially automated procedure. The provided systems and methods use image processing and image-generated topological skin models.

The present invention, that is a multi-channel opening device, is advantageous as it eases the opening of hair channels, which is extremely exhausting and requires high concentration. With the present invention, extra incision is not made unnecessarily when opening the hair channels. Thanks to the angle and directional characteristics of the multi-channel opening device, the direction (right and left tilt) and angle (horizontal or perpendicular) of the hair channels that are opened to achieve natural results in hair transplantation can be determined. In addition, it does not damage the natural hair follicles and it can be counted how many hair channels are opened to the right and left parts of the head.

The structural and characteristic features and all advantages of the invention will become apparent from the following detailed description and the detailed description. For this reason, this assessment should be made by considering these figures and detailed explanation.

### Figures to help explain the invention

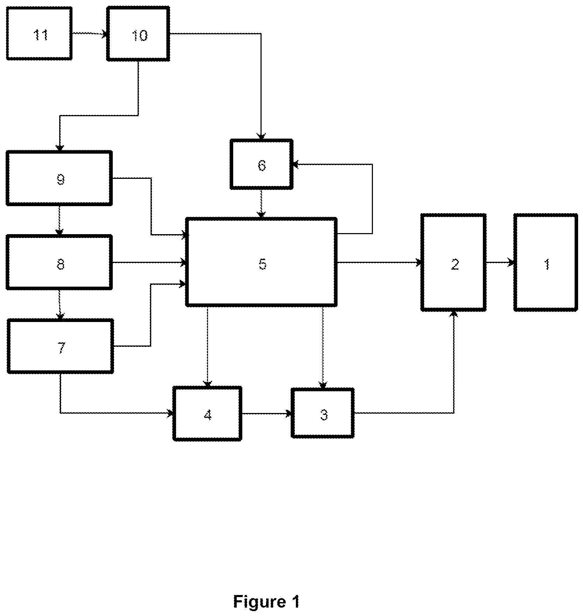

Figure 1: Overview of system components of a multi-hair channel opening device

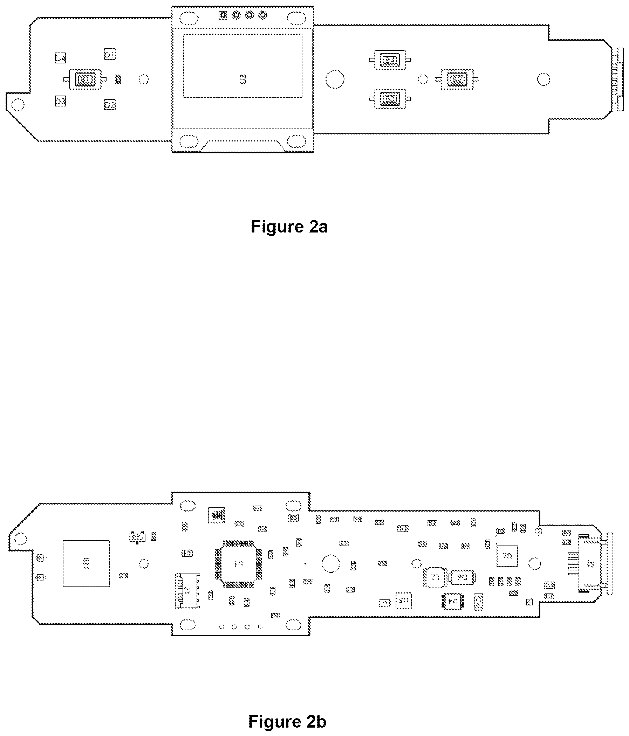

Figure 2a: Top view of the circuit board of the multi-hair channel opening device
Figure 2b: Bottom view of the circuit board of the multi-hair channel opening device
Figure 3a: Front view of 2-point micro- blades for multi-hair channel opening device
Figure 3b: Side pattern of 2-point micro blades for multi-channel opening device
Figure 3c: Front view of 3-point micro- blades for multi-channel opening device
Figure 3d: Side view of 3-point micro- blades for multi-channel opening device
Figure 4a: Front view of multi-hair channel opening device
Figure 4b: Side view of the multi-hair channel opening device
Figure 4c: Rear view of the multi-hair channel opening device

### References to help explain the invention

**1.** Linear motor
**2.** Motor driver
**3.** Laser
**4.** Audible stimulus
**5.** Micro processor
**6.** Digital display
**7.** Acceleration and protractor
**8.** Buttons
**9.** Battery reader
**10.** Power center
**11.** Ion battery

### Detailed explanation of the Invention

The multi-hair channel opening device which is the subject of the invention is described in detail below, with no limiting effects.

Our invention as defined in claim 1, the multi-channel opening device, consists of the following apparatus:

linear motor (1); motor driver (2), laser (3), buzzer (4), microprocessor (5), digital display (6), accelerometer and protractor (7), buttons (8), battery reader (9), power station ( 10) consists of an ion battery (11) and micro blades inserting apparatus.

The linear motor (1) is the motor which performs the reverse motion of the multi-hair channel- opening device in the incision opening process in the light of the data from the microprocessor (5). In addition, it performs the channel- opening procedure of the engine at the automatically adjusted depth in the light of the information processed by the acceleration and the gauge (7). By reducing the workload of the physician in the manual method, it helps the physician to concentrate more on the aesthetic part in the hair ducts. At the same time, thanks to the linear motor (1), the number of opening channels can be counted automatically.

The motor drive (2) is the unit that interprets the required voltage and current values of the motor that makes the channel- opening procedure of the multi-channel opening device from the microprocessor (5).

With the laser integrated in the device (3), the part where the device will make cuts is displayed before opening the channels. The laser is the unit that allows the area of the channel to be marked before opening the channel. Only the laser (3) is used for the purpose of the indicator with the light source. In this way, the channels do not overlap. The incision on the existing healthy hair follicles is prevented and thus healthy hair follicles are not damaged during the channel opening procedure.

The audible buzzer (4) is the unit that emits a sound signal that emits and directs the user by making a beep for the transitions between the menus of the multi-hair channel opening device.

The microprocessor (5) is the central unit in which all functions are processed. Interpret the information from the acceleration and angle meter (7) and display the display on the digital display (6). At the same time, the commands required for the linear motor (1), the buzzer (4) and the laser (9) are transmitted from this unit.

With the present invention, physicians may be familiar with the channels opened momentarily with the digital display (6). The number of channels opened on the digital display (6), direction and angle information of the device, and the lithium ion battery (11) energy level can be monitored instantly. The digital display is the part where the multi-hair channel opening device will interact with the user.

The acceleration and angle meter (7) is the unit that reads the current angle and direction of the multi-hair channel opening device. The information read from here is transmitted to the microprocessor (5).

Buttons (8) are the parts that allow various settings of the multi-hair channel opening device (incision speed, tip change, number of knives to change etc.). At the same time, a single button (8) is assigned for channel- opening procedure. For the settings part, 3 (optionally 4) buttons (8) are separated.

The battery reader (9) is the unit that reads the current voltage level of the lithium-ion battery (11) to the microprocessor (5).

Power center (10), energy from the lithium ion battery (11), microprocessor (5), digital display (6), buttons (8), acoustic warning (4), acceleration and protractor (7), motor drive (8) and linear converts it to the appropriate voltage level.

The ion battery (11) is the power supply to which all the energy of the multi-hair channel- opening device is provided. Lithium ion battery (11) was used.

The device does not lose its angle in case of movement of the patient or physician. In case of changing the angle of the device, the end of the device moves and calibrates automatically so that the channel to be opened remains at the same depth. If the patient or the physician moves with the digital display (6), the device does not lose its direction. When the patient's hair is lengthened, how many degrees of hair can be extended to the right or left can be determined by opening the hair ducts. In addition, when opening the hair channels, the depth can be set in mm from the digital display (6).

The present invention, in the multi-hair channel opening device, sticks the microprocessors that open the hair ducts. It can hold more than one knife at the same time. Thanks to the micro blades, the blades can be opened very close to the blades or the blades can be opened very frequently and the channels can be opened by increasing the distance between the blades.

In the case of multiple blades placed in the micro blades insertion apparatus, the blades remain parallel to each other, but in the position defined as a zigzag or staircase. In this way, instead of opening the channels completely side by side to get the result of natural hair transplantation is provided to open in a certain distribution. When it comes together with the properties of the blades, the special geometric makes an incision in the figurines. This device can also be used as a single knife and has the option of fixing several knives in a parallel and parallel way.

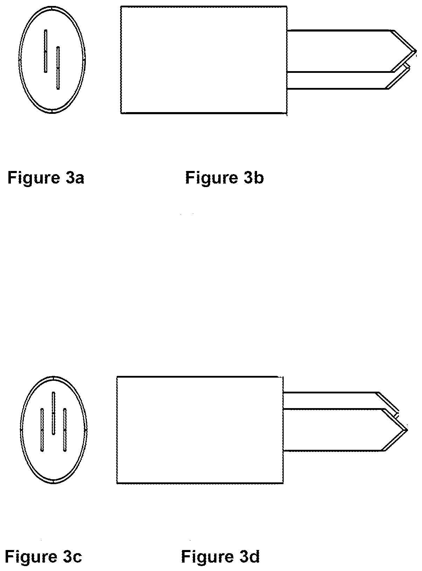

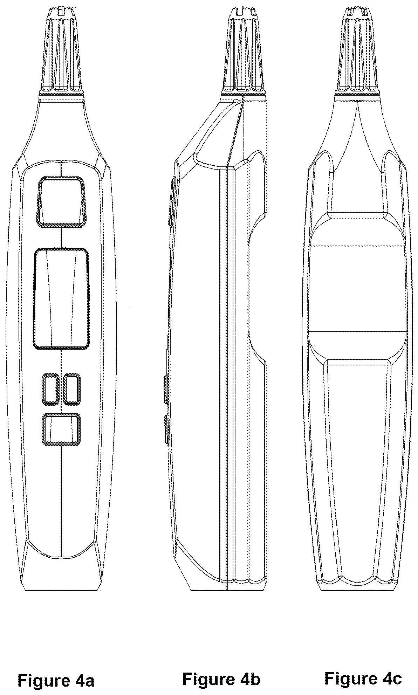

As shown in Figures 3a-3b-4a-4b, in the present invention, the angle difference between the blades is provided in order to allow the blades to enter the skin evenly in the angled incision opening process. In this way, the incisions made at the previously determined angles and that are open in the skin are provided to have an equal depth.

In the present invention there are different speed modes for opening the hair channels. The user can perform the operation in a slow or medium mode or in faster mode. The device can also operate manually by disabling all functions and can only perform channel counting in manual mode. With this multi-hair channel- opening device, it is possible to plan very frequent (dense), medium or infrequent hair transplantation when opening the channels during hair transplantation, according to physician's request.

It is evident that a skilled person in the art will also be able to demonstrate the novelty set forth in the invention by using similar methods and / or apply this method to other similar purposes as used in the art. Thus, it is obvious that such methods will not be deemed to be novelty and in particular will be deprived of the criteria for exceeding the state of the art.

## Claims

1. A multi-channel opening device which can be used to achieve natural results in hair transplantation, can be manually operated with speed modes, all functions can be deactivated, and with which it is possible to make very frequent, middle or infrequent hair transplantation planning, the features of the invention are as follows;
• central unit microprocessor (5) that processes all functions, interprets the information from an accelerometer and protractor (7) on the digital display, as well as transmitting commands to a linear motor (1), buzzer (4), laser (9),
• digital display (6), which allows physicians to have instant information about opened channels, number of opened channels, direction, depth and angle information of the device, energy level of a lithium ion battery (11), which can be monitored instantly and interact with the user, and
• wherein the linear motor (1) performs back and forth movement of the incision in the light of the data coming from the microprocessor (5), which automatically performs the channel- opening procedure of the engine at the depth set in the engine in light of the information processed by the acceleration and protractor (7),
• a motor driver (2) which interprets and performs the necessary voltage and current values of the motor that makes the movements of the channel- opening procedure process, and the information coming from the microprocessor (5),
• the laser (3) is integrated into the device that allows the device to show the part of the device where the cut is to be taken before the channel is opened and to mark the location of the channel before opening the channel,
• the audible buzzer (4) emits a signal that emits and directs the user by making an audible signal when switching between their menus,
• the accelerometer (7) reads the instantaneous angle and direction and transmits this readings to the microprocessor (5),
• single buttons (8) for channel- opening procedure, which allow various settings (cut speed, change of tip, change of knife number, etc),
• battery reader (9) for reading the current voltage level of the lithium-ion battery (11) to the microprocessor (5),
• the power center (10) that converts the energy coming from the ion battery (11) to the voltage level corresponding to the microprocessor (5), digital display (6), push buttons (8), buzzer (4), acceleration and protractor (7), motor drive (8) and the linear motor (1),
• the ion battery (11) is the power source of all energy,
• further comprising means for fixing micro-blades opening hair channels, that can hold more than one blade at the same time that can perform very frequent channel opening device by holding the blades very close, that can be opened at a medium frequency by increasing the distance between the blades, that has blades standing parallel to each other but standing in a position that defines it as a zigzag or staircase, and that it is a device including parts of a micro-blade insert with a single-blade or multiple-blade fixing option, which can be cut in a special geometric shape.

2. The multi-hair channel opening device according to claim 1, the device is **characterized in that** the end of the device is automatically calibrated; so that the channel to be opened will remain at the same depth so that the device will not lose its angle and changes the device angle in case the patient or physician moves.

## Patentansprüche

1. Eine Mehrkanal-Öffnungsvorrichtung, die verwendet werden kann, um natürliche Ergebnisse bei der Haartransplantation zu erzielen, die manuell mit Geschwindigkeitsmodi betrieben werden kann, bei der alle Funktionen deaktiviert werden können, und mit der es möglich ist, eine sehr häufige, mittlere oder seltene Haartransplantationsplanung durchzuführen, die Merkmale der Erfindung sind wie folgt;
Mikroprozessor-Zentraleinheit (5), die alle Funktionen verarbeitet, die Informationen eines Beschleunigungs- und Winkelmesser (7) auf dem digitalen Display interpretiert und Befehle an einen Linearmotor (1), einen Summer (4) und einen Laser (9) übermittelt,
- Digitalanzeige (6), die dem Arzt sofortige Informationen über die geöffneten Kanäle, die Anzahl der geöffneten Kanäle, die Richtung, die Tiefe und den Winkel des Geräts, den Energiestand einer Lithium-Ionen-Batterie (11), der sofort überwacht werden kann, liefert
wobei der Linearmotor (1) die Hin- und Herbewegung des Einschnitts im Lichte der vom Mikroprozessor (5) kommenden Daten durchführt, der automatisch den Kanalöffnungsvorgang des Motors in der im Motor eingestellten Tiefe im Lichte der vom Beschleunigungs- und Winkelmesser (7) verarbeiteten Informationen durchführt,
einen Motortreiber (2), der die erforderlichen Spannungs- und Stromwerte des Motors, der die Bewegungen des Kanalöffnungsvorgangs ausführt, und die vom Mikroprozessor (5) kommenden Informationen interpretiert und ausführt,
der Laser (3) ist in das Gerät integriert, der es dem Gerät ermöglicht, den Teil des Geräts anzuzeigen, an dem der Schnitt vor dem Öffnen des Kanals vorgenommen werden soll, und die Stelle des Kanals vor dem Öffnen des Kanals zu markieren,
- der akustische Summer (4) gibt ein Signal ab, das den Benutzer durch ein akustisches Signal beim Umschalten zwischen den Menüs anweist,
- Der Beschleunigungs- und Winkelmesser (7) misst den momentanen Winkel und die Richtung und überträgt diese Werte an den Mikroprozessor (5),
- einzelne Tasten (8) zur Kanalöffnung, die verschiedene Einstellungen ermöglichen (Schnittgeschwindigkeit, Wechsel der Spitze, Änderung der Messernummer usw.),
Batterieleser (9) zum Ablesen des aktuellen Spannungspegels der Lithium-Ionen-Batterie (11) an den Mikroprozessor (5),
das Leistungszentrum (10), das die von der Ionenbatterie (11) kommende Energie in die dem Mikroprozessor (5) entsprechende Spannung umwandelt, die Digitalanzeige (6), die Drucktasten (8), den Summer (4), den Beschleunigungs- und Winkelmesser (7), den Motorantrieb (8) und den Linearmotor (1),
- die Ionenbatterie (11) ist die Energiequelle für die gesamte Energie,
- weiter umfassend Mittel zum Befestigen von Mikroklingen, die Haarkanäle öffnen, die mehr als eine Klinge gleichzeitig halten können, die eine sehr häufige Kanalöffnungsvorrichtung durchführen können, indem sie die Klingen sehr nahe halten, die mit einer mittleren Frequenz geöffnet werden können, indem der Abstand zwischen den Klingen vergrößert wird, die Klingen haben, die parallel zueinander stehen, aber in einer Position stehen, die sie als Zickzack oder Treppe definiert, und dass es eine Vorrichtung ist, die Teile eines Mikroklingeneinsatzes mit einer Einzelklingen- oder Mehrfachklingenbefestigungsoption enthält, die in einer speziellen geometrischen Form geschnitten werden kann.

2. Die Vorrichtung zum Öffnen von Kanälen mit mehreren Haaren nach Anspruch 1, wobei die Vorrichtung **dadurch gekennzeichnet ist, dass** das Ende der Vorrichtung automatisch kalibriert wird, so dass der zu öffnende Kanal in der gleichen Tiefe bleibt, so dass die Vorrichtung ihren Winkel nicht verliert und den Winkel der Vorrichtung ändert, wenn sich der Patient oder der Arzt bewegt.

## Revendications

1. Il s'agit d'un dispositif d'ouverture à canaux multiples qui peut être utilisé pour obtenir des résultats naturels dans la transplantation de cheveux, il peut être utilisé manuellement, il a des modes de vitesse, toutes les fonctions peuvent être désactivées, il est possible de planifier une transplantation de cheveux très fréquente, moyenne ou clairsemée, les caractéristiques du dispositif sont les suivantes :
Une unité centrale de microprocesseur (5) qui traite toutes les fonctions, interprète les informations provenant de l'accéléromètre et du gyroscope (7) sur un écran numérique, et transmet les commandes au moteur linéaire (1), à la sonnerie (4), et au laser (9),
Un écran numérique (6) qui permet aux médecins d'obtenir des informations en temps réel sur les canaux ouverts, le nombre de canaux ouverts, les informations de direction, de profondeur et d'angle de l'appareil, ainsi que le niveau d'énergie d'une batterie lithium-ion (11) pouvant être surveillée en temps réel
Dans ce cas, le moteur linéaire (1) effectue le mouvement alternatif de l'incision à la lumière des données fournies par le microprocesseur (5) et exécute automatiquement la procédure de rainurage du moteur à la profondeur définie dans le moteur à la lumière des informations traitées par l'accéléromètre et le rapporteur (7),
C'est un pilote de moteur (2) qui interprète et réalise les valeurs de tension et de courant requises pour le moteur et les informations provenant du microprocesseur (5) qui exécute les mouvements de l'opération de rainurage,
Le laser (3) est intégré à l'appareil et permet à ce dernier de montrer la partie de l'incision et de marquer l'emplacement du canal avant d'ouvrir le canal,
- l'avertisseur sonore (4) émet un signal sonore pour guider l'utilisateur lorsqu'il passe d'un menu à l'autre.
- L'accéléromètre et le rapporteur (7) lisent l'angle et la direction instantanés et transmettent ces données au microprocesseur (5).
- des boutons uniques (8) pour la procédure d'ouverture du canal, qui permettent divers réglages (vitesse de coupe, changement de pointe, changement du nombre de couteaux, etc,)
Lecteur de batterie (9) pour lire le niveau de tension actuel de la batterie lithium-ion (11) et le transmettre au microprocesseur (5),
Le centre d'alimentation (10) qui convertit l'énergie provenant de la batterie ionique (11) au niveau de tension correspondant au microprocesseur (5), à l'affichage numérique (6), aux boutons poussoirs (8), au buzzer (4), à l'accélération et au rapporteur (7), le moteur d'entraînement (8) et le moteur linéaire (1),
- La batterie ionique est la source de toute énergie,
- En outre, il comprend des moyens de fixation des micro-lames qui ouvrent les canaux capillaires, pouvant contenir plus d'une lame à la fois, permettant d'ouvrir très fréquemment les canaux en tenant les lames très près les unes des autres, pouvant s'ouvrir à une fréquence moyenne en augmentant la distance entre les lames, que ses lames sont parallèles les unes aux autres, mais dans une position qui les définit comme des zigzags ou des escaliers. Et qu'il s'agit d'un dispositif comprenant des parties d'un élément de micro-lame avec une option de fixation à une ou plusieurs lames, qui peut être découpé dans une forme géométrique spéciale.

2. L'invention concerne un dispositif d'ouverture de canal à plusieurs cheveux selon la revendication 1, le dispositif étant **caractérisé par le fait que** l'extrémité du dispositif est automatiquement calibrée, de sorte que le canal à ouvrir reste à la même profondeur, que le dispositif ne perde pas son angle et qu'il ne change pas d'angle, en cas de mouvement du patient ou du médecin.
